# EUROPEAN PATENT SPECIFICATION

(11) **EP 0 681 584 B1**
(45) Date of publication and mention of the grant of the patent: **06.06.2001**
(21) Application number: 94906611.2
(22) Date of filing: 18.01.1994
(51) Int. Cl.: C07D 513/04, A61K 31/54

(54) **CONDENSED HETEROCYCLIC GLUTAMIC ACID DERIVATIVES AS ANTIPROLIFERATIVE AGENTS**
KONDENSIERTE HETEROCYCLISCHE GLUTAMINSÄUREDERIVATE ALS ANTIPROLIFERATIVE MITTEL
DERIVES D'ACIDE GLUTAMIQUE HETEROCYCLIQUE CONDENSE UTILISES EN TANT QU'AGENTS ANTIPROLIFERATIFS

(30) Priority: 29.01.1993 US 10861
(43) Date of publication of application: 15.11.1995
(60) Divisional application: 00122564.8
(73) Proprietor: AGOURON PHARMACEUTICALS, INC., San Diego, CA 92121 (US)
(72) Inventor: VARNEY, Michael, D., Carlsbad, CA 92009 (US); PALMER, Cynthia, L., LeMesa, CA 91941 (US); ROMINES, William, H., San Diego, CA 92130 (US)
(74) Representative: Grünecker, Kinkeldey, Stockmair & Schwanhäusser Anwaltssozietät
(86) International application number: US9400418
(87) International publication number: WO9417076

(56) References cited:
- EP-A- 0 438 261
- WO-A-86/05181
- CHEMICAL ABSTRACTS, vol. 113, no. 9, 27 August 1990, Columbus, Ohio, US; abstract no. 70792k, R. L. KISLIUK ET AL 'Antitumor, cytotoxic, and enzyme inhibitory properties of homofolates' page 29 ; & MOL. ASPECTS CHEMOTHER., PROC. INT. SYMP., 1988 (PUB. 1990)
- CHEMICAL AND PHARMACEUTICAL BULLETIN. vol. 40, no. 1 , January 1992 , TOKYO JP pages 49 - 52 M. SAKO ET AL 'First synthesis of 5,6,7,8-tetrahydro-8-deaza-8-thiafolic acid'

## Description

The present invention relates to certain compounds, defined below, which inhibit the enzyme glycinamide ribonucleotide formyl transferase (GARFT), to intermediate compounds, to pharmaceutical compositions containing the certain compounds, to the use thereof to inhibit GARFT, and to the use thereof to inhibit the growth and proliferation of the cells of higher organisms or microorganisms such as bacteria, yeast and fungi. Such effects include antitumor, antiinflammatory, antipsoriatic and immunosuppressive activity. A process for the preparation of these compounds is also disclosed.

The large class of antiproliferative agents includes antimetabolite compounds. A particular subclass of antimetabolites known as antifolates or antifoles are antagonists of the vitamin folic acid. Typically, antifolates closely resemble the structure of folic acid and incorporate the characteristic P-benzoyl glutamate moiety of folic acid. The glutamate moiety of folic acid takes on a double negative charge at physiological pH. Therefore, this compound and its analogs have an active energy driven transport system to cross the cell membrane and exert a metabolic effect.

Glycinamide ribonucleotide formyl transferase (GARFT) is a folate dependent enzyme in the *de novo* purine biosynthesis pathway. This pathway is critical to cell division and proliferation. Shutting down this pathway is known to have an antiproliferative effect, in particular, an antitumor effect. Thus, a number of folate analogs have been synthesized and studied for their ability to inhibit GARFT. A prototypic specific tight binding inhibitor of GARFT, 5,10-didedzatetrahydrofolic acid, has been reported to show antitumor activity. See F.M. Muggia, "Folate antimetabolites inhibitory to de novo purine synthesis" in New Drugs, Concepts and Results in Cancer Chemotherapy, pp.65-87, Kluwer Academic Publishers, Boston (1992).

EP-A-438261 discloses a compound of the formula (A) having antitumor activity 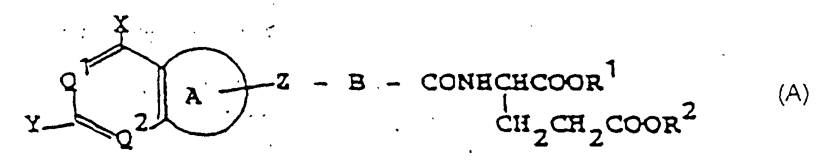 wherein
the ring A is a 5-membered ring, provided that, in the case where the ring A is a pyrrole or pyrroline ring, (i) the N atom in the ring has no active proton or (ii) the ring bonds to Z at the -position of the ring ;
B is a divalent cyclic or chain group which may be substituted; one of Q¹ and Q² is N, with the other being
N or CH ; Y is a hydrogen or halogen atom or a group which bonds to the bonding line with a carbon, nitrogen, oxygen or sulfur atom ; X is an amino, hydroxyl or mercapto group ;
Z is a straight-chain divalent group having a number of atoms of 2 to 5 composed of carbon atoms which each may be substituted, or of carbon atoms which each may be substituted and one hetero atom which may be substituted ;
-COOR¹ and -COOR² each is the same as or different from the other and represents a carboxyl group which may be esterified or its salt.

It is the object of the present invention to provide a novel class of compounds containing a glutamic acid which compounds are effective in inhibiting the enzyme glycinamide ribonucleotide formyl transferase (GARFT) and the growth and proliferation of cells of higher organisms and of microorganisms such as bacteria, yeast and fungi. It is a further object of the invention to provide pharmaceutical compositions containing these compounds or suitable salts thereof and to use these compounds as inhibitors of the enzyme GARFT.

According to the present invention, the object is attained by a compound of the formula (I): 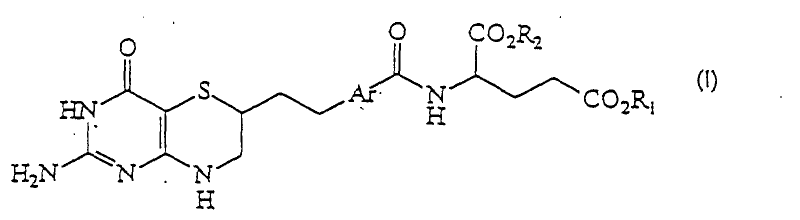 wherein
Ar is a monocyclic carbocyclic or heterocyclic ring;
   and
R₁ and R₂ are independently hydrogen or a C₁₋₆ alkyl group;
   or a pharmaceutically acceptable salt thereof.

In formula (1), Ar is preferably phenylene.

Particularly preferred moieties R₁ and R₂ are hydrogen and C₂ alkyl.

The compounds of the present invention are preferably optically active.

The present invention further provides an antiproliferative composition comprising the compound of formula (I) in combination with a pharmaceutically acceptable carrier.

According to the present invention, the compound of formula (I) can be used for the preparation of a medicament for inhibiting the growth and proliferation of the cells of microorganisms or of higher organisms in a host.

Furthermore, the compound of formula (I) can be used according to the present invention for the preparation of a medicament for inhibiting GARFT.

The compounds of the invention possess anti- proliferative activity, a property which may express itself in the form of anti-tumor activity. A compound of the invention may be active *per se,* or it may be a precursor which is converted *in vivo* to an active compound. Preferred compounds of the invention are active in inhibiting the enzyme GARFT. Particularly preferred compounds are active in inhibiting the growth of the L1210 cell line, a mouse leukemia cell line which can be grown in tissue culture. Compounds of the invention should also be active in inhibiting the growth of bacteria such as *Escherichia coli* gram negative bacteria which can be grown in culture.

The compounds according to the invention, as well as the pharmaceutically acceptable salts thereof, may be incorporated into convenient dosage forms such as capsules, tablets, or injectable preparations. Solid or liquid pharmaceutically acceptable carriers may also be employed. Solid carriers include starch, lactose, calcium sulphate dihydrate, terra alba, sucrose, talc, gelatin, agar, pectin, acacia, magnesium stearate and stearic acid. Liquid carriers include syrup, peanut oil, olive oil, saline solution and water.

The carrier or diluent may include any prolonged release material, such as glyceryl monostearate or glyceryl distearate, alone or with wax. When a liquid carrier is used, the preparation may be in the form of a syrup, elixir, emulsion, soft gelatin capsule, sterile injectable liquid (e.g. solution) or a nonaqueous or aqueous liquid suspension.

The pharmaceutical preparations are prepared following conventional techniques of the pharmaceutical chemist involving steps such as mixing, granulating, and compressing when necessary for tablet forms, or mixing, filling and dissolving the ingredients as appropriate to give the desired products for oral, parenteral, topical, intravaginal, intranasal, intrabronchial, intraocular, intraaural and rectal administration.

The compositions of the invention may further comprise one or more other compounds which are anti-tumor agents such as: a mitotic inhibitor (for example: vinblastine); alkylating agents; dihydrofolate reductase inhibitors or TS inhibitors; antimetabo-lites (for example, 5-fluorouracil and cytosinearabinoside); intercalating antibiotics (for example, adriamycin and bleomycin); enzymes (for example, asparaginase); topoisomerase inhibitors (for example, etoposide); biological response modifiers (for example, interferon); or the compounds described in copending and commonly assigned U.S. patent application no. 07/991,259, filed December 16, 1992.

The compositions of the invention may also comprise one or more other compounds including antibacterial, antifungal, antiparasitic, antiviral, antipsoriatic and anticoccidial agents. Exemplary antibacterial agents include, for example, sulfonamides such as sulfamethoxazole, sulfadiazine, sulfameter or sulfadoxine; dihydrofolic reductase inhibitors such as trimethoprim, bromodiaprim, or trimetrexate; penicillins; cephalosporins; aminoglyclosides; bacteriostatic inhibitors of protein synthesis; the quinolone carboxylic acids and their fused isothiazolo- analogs.

According to the invention the compounds of formula (I) are used in a therapeutic process of inhibiting the growth and proliferation of cells of higher organisms or microorganisms which comprises administering to a host an effective amount of a compound according to the present invention. The compounds of the invention are particularly useful in the treatment of mammalian hosts such as human hosts and in the treatment of avian hosts. A particularly preferred therapeutic process comprises administering to a host an effective amount of a compound according to the present invention to inhibit GARFT.

Many of the antiproliferative drugs described herein or pharmaceutically acceptable salts thereof can be employed in the therapeutic process of the invention. The compounds may be administered in the form of a pharmaceutically acceptable composition comprising a diluent or carrier such as those described above.

Doses of the compounds preferably include pharmaceutical dosage units comprising an effective quantity of the active compound. An "effective quantity" means a quantity sufficient to inhibit the folate metabolic pathways and derive the beneficial effects therefrom through administration of one or more of the pharmaceutical dosage units.

An exemplary daily dosage unit for a vertebrate host comprises an amount up to one gram of active compound per kilogram of the host, preferably one half gram, more preferably 100 milligrams, and most preferably, about 50 milligrams or less per kilogram of the host weight. The selected dose may be administered to a warm-blooded animal or mammal, for example, a human patient in need of treatment mediated by folate metabolic pathways inhibition, by any known method of administrating the dose including topically as, for example, an ointment or cream; orally; rectally, for example, as suppository; parenterally by injection; or continuously by intravaginal, intranasal, intrabronchial, intraaural or intraocular infusion.

The compounds according to the present invention may be characterized as producing any one or more of an antiproliferative effect, an antibacterial effect, an antiparasitic effect, an antiviral effect, an antipsoriatic effect, an antiprotozoal effect, an anticoccidial effect, an antiinflammatory effect, an immunosupressive effect or an antifungal effect. The compounds are especially useful in producing an antitumor effect in a vertebrate host harboring a tumor.

The present invention relates to antiproliferative compounds capable of inhibiting GARFT and having the above formula (I)

Although the compounds are depicted in the formula (I) in the 4-oxo form and are referred to as such throughout this description, the oxo group exists in tautomeric equilibrium with the corresponding 4-hydroxy group and it will be understood that in each case the tautomeric hydroxyl form is also indicated.

The compounds of formula (I) in which each of R₁ and R₂ is hydrogen are active anti-tumor and antiproliferative compounds. The compounds of formula (I) wherein R₁ and R₂ are moieties which form a readily hydrolyzable ester group, namely a C₁₋₆ alkylgroup, preferably an ethyl group, are novel intermediates for forming the free glutamic acid forms of the compounds and can also be hydrolyzed *in vivo* and thus act as prodrugs.

The invention also includes pharmaceutically acceptable salts, including, for example, alkaline metal, alkaline earth metal, other non-toxic metals, ammonium and substituted ammonium salts of the glutamic acid embodiments of the invention such as, but not limited to, the sodium, potassium, lithium, calcium, magnesium, aluminum, zinc, ammonium, trimethyl ammonium, triethyl ammonium, pyridinium and substituted pyridinium salts.

Novel compounds of this invention can be prepared by reacting a compound having the formula (III). 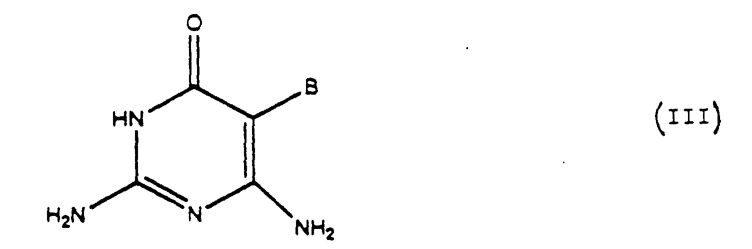 wherein:
B is a halogen, preferably bromo, with a compound having the formula (IV) 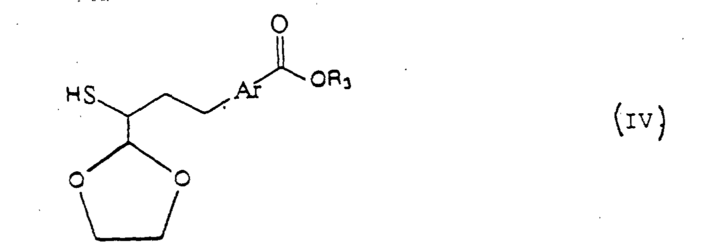 wherein Ar is as defined in formula (I) and R₃ is hydrogen or a straight, branched or cyclic C₁ to C₆ alkyl group optionally carrying one or more halogen, hydroxyl, or amino groups; in the presence of base other than the compound of formula (III), preferably a non-nucleophilic auxiliary base, in a solvent in which at least one of the reactants is at least partially soluble under conditions sufficient to obtain the compound of formula(V) 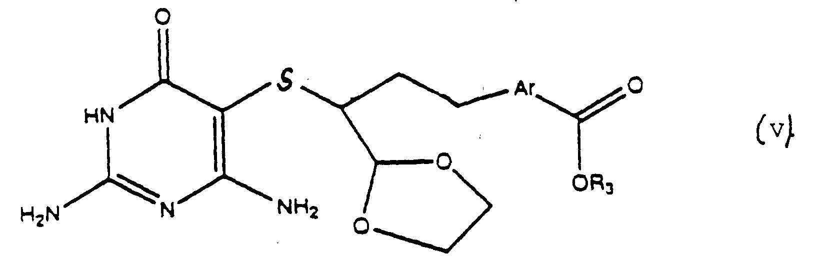 wherein Ar is defined as in formula (I) and R₃ is as defined in formula (IV).

The reaction of compound (III) with compound (IV) is preferably carried out in a suitable solvent in which at least one or both reactants are soluble at the reaction temperature. The solvent and the reaction environment are preferably purged of oxygen prior to introduction of the reactants by bubbling an inert gas, such as argon or nitrogen, through the solvent. Bubbling of the inert gas is preferably continued until the reaction has gone to completion and been quenched, such as by pouring into water. Suitable preferred solvents are dipolar aprotic solvents such as, e.g., dimethylsulfoxide, N,Ndimethylformamide, N,N-dimethylacetamide, or N-methyl-2-pyrrolidinone.

The basic medium for the reaction of compounds (III) and (IV) is preferably provided via a non-nucleophilic auxiliary base which is defined as a base capable of neutralizing hydrogen halide gas generated by the substitution reaction. The base is preferably an alkali or alkali earth metal carbonate or a trialkylamine such as, e.g., trimethylamine, triethylamine or diisopropylethylamine.

The preferred method for conducting the reaction of compounds of the formulae (IV) and (III) is to suspend the compound of formula (III), preferably, 5-bromo-2,6-diamino-4(3H)-oxo-pyrimidine, in the solvent; compound of the formula (IV) and the auxiliary base are then added sequentially.

The reaction vessel can then immersed in an oil bath which has been heated to the appropriate temperature (20-200°, preferably 70-120°C). The reaction mixture can be stirred at this temperature for the requisite length of time (usually 30-330 minutes), then followed by cooling to room temperature and pouring into water. The product, the compound of formula (V), can then be isolated by filtration or extraction with an organic solvent and purified either by recrystallization or by chromatography.

The compound of formula(V)can be reacted with an acid, preferably hydrochloric acid, in a suitable solvent, preferably tetrahydrofuran, under conditions sufficient, preferably reflux, to obtain a compound of the formula (VI) 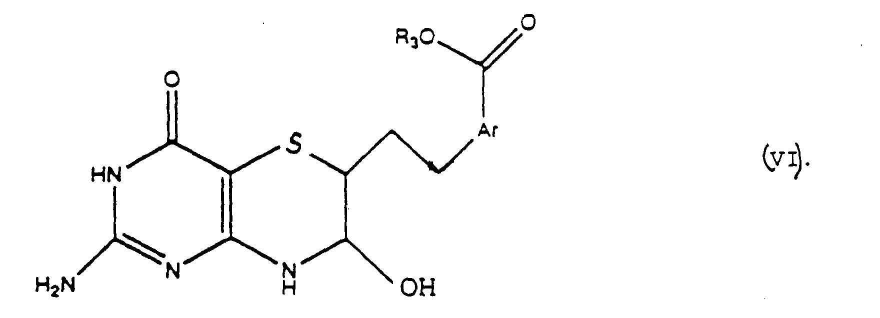 wherein Ar is defined as in formula (I) and R₃ is as defined in formula (IV).

Compound (VI) is then reduced, preferably with sodium cyanoborohydride, to obtain a compound of the formula (VII) 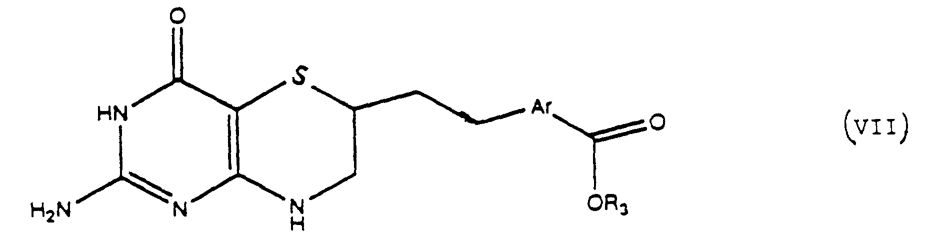 wherein Ar is defined as in formula (I) and R₃ is as defined in formula (IV).

The compound of formula (VII) is hydrolyzed, under basic conditions, to form a compound of the formula (VIII) 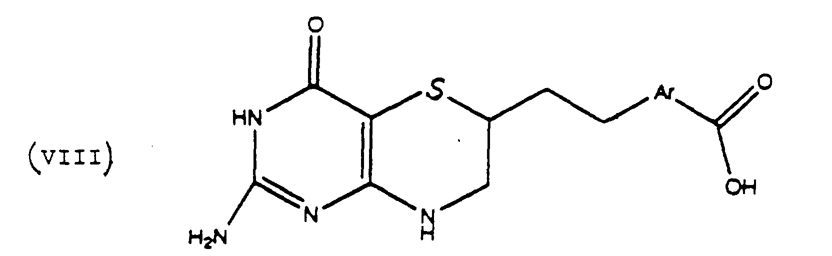 wherein Ar is defined as in formula (I). If, in the compound of formula (VII), R₃ is hydrogen, then this reaction step is not necessary, and the compound of formula VII can be peptide coupled, as described immediately below.

The compound of formula (VIII) (or the compound of formula (VII), wherein R₃ is hydrogen), which is in free carboxylic acid form, can be peptide coupled, by means well-known to those skilled in the art, with a glutamic acid diester hydrochloride to form a diester of the formula (IX) 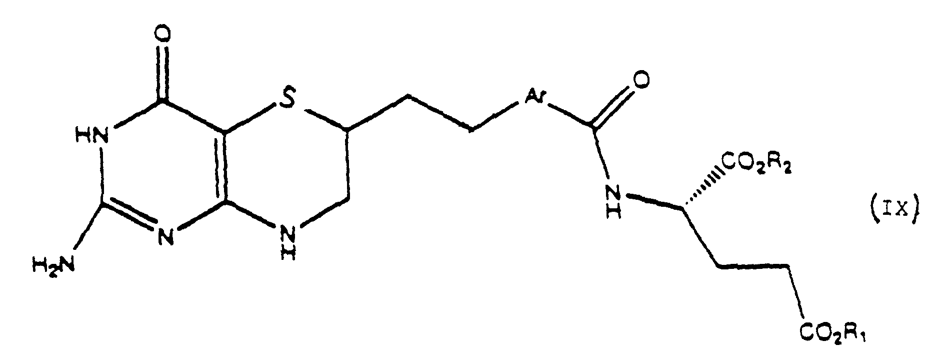 wherein Ar, R₁ and R₂ are as defined in formula (I), with the proviso that neither R₁ nor R₂ is hydrogen.

Finally, if desired, the compound of formula (IX) can be hydrolyzed to the free glutamic acid form depicted in formula (I) (R₁ and R₂ = H). A detailed synthesis for compounds within formula (I) will be presented in the examples that follow.

Specific examples of novel compounds of formula (I) include:
(2-[4-[2-(2-Amino-4-oxo-4,6,7,8-tetrahydro-3H-pyrimido[5,4-b][1,4]thiazin-6-yl)-ethyl]-benzoylamino]-pentanedioic acid diethyl ester); and (2-[4-[2-(2-Amino-4-oxo-4,6,7,8-tetrahydro-3H-pyrimido[5,4-b][1,4]thiazin-6-yl)-ethyl]benzoylamino]-pentanedioic acid). The d-form of these compounds is preferred.

It is to be understood that application of the teachings of the present invention to a specific problem or environment will be within the capabilities of one having ordinary skill in the art in light of the teachings contained herein. Examples of the products of the present invention and a representative process for their preparation and recovery appear in the following examples.

### EXAMPLE 1

### Step 1a

### (1) (1,1-Dimethoxy-but-3-yn-2-ol)

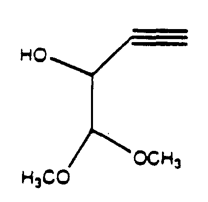

To a stirred solution of 1.037 g (10.55 mmol) of trimethylsilyl acetylene in 50 ml of dry tetrahydrofuran (THF) under argon at -78°C was added dropwise 6.6 ml of 1.6 M n-butyl lithium. After 10 minutes at -78°C, a solution of 1.21 g (10.46 mmol) of glyoxal dimethyl acetal in 5 ml of tetrahydrofuran (THF) was added dropwise. After 1 hour at -78°C, the reaction was quenched with about 1 ml of H₂O and allowed to warm to room temperature, diluted with ethyl acetate and washed with saturated NaCl solution. The aqueous layer was reextracted with ethyl acetate and combined organic layers were dried over MgSO₄ and concentrated under reduced pressure. To 785 mg of the resulting yellow oil dissolved in tetrahydrofuran (THF) was added 5.8 ml of 1.0 M tetrabutylammonium fluoride in tetrahydrofuran (THF). After heating for 1 hour at 50°C, the volatiles were evaporated and the residue was flash chromatographed on silica eluting methylene chloride/ethyl acetate (9:1). In this manner, there was obtained 412 mg (60% overall) of the alkyne-alcohol, compound (1), as a colorless oil.
IR (neat) 3441 (broad), 3277, 2944, 2839, 1636, 1450, 1196, 1084cm⁻¹.
¹H NMR (CDCl₃) δ 2.42 (bs,1H), 2.49 (s,1H), 3.51 (s,3H), 3.53 (s,3H), 4.36 (bs,2H).
Analysis calculated for C₆H₁₀O₃ · 0.35 H₂O; C, 52.81: H, 7.90.
Found: C, 52.87; H, 7.86.

### Step 1b

### (2) (4-(3-Hydroxy-4,4-dimethoxy-but-1-ynyl)-benzoic acid methyl ester)

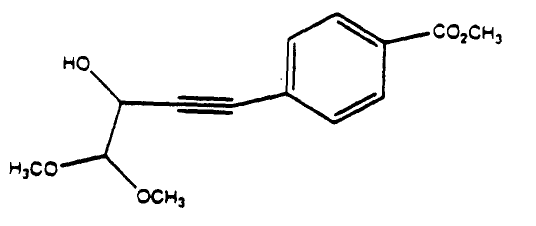

To a stirred solution of 232 mg (1.78 mmol) of the alkyne compound (1) and 467 mg (1.78 mmol) of Methyl-4-Iodobenzoate in 5 ml diethylamine was added 13 mg (0.18 mmol) of Bis(triphenylphosphine) palladium(II)chloride and 7mg (.036 mmol) of cuprous iodide. After 15 hours at room temperature, volatiles were removed under reduced pressure and the residue was flash chromatographed on silica eluting methylene chloride/ethyl acetate (12:1). In this manner, there was obtained 347 mg (74%) of the compound (2) as an orange oil.
IR (neat) 3451 (broad), 2953, 2838, 1717, 1607, 1437, 1310, 1283, 1120, 1082cm⁻¹.
¹H NMR (CDCl₃) δ 2.46 (bs,1H), 3.55 (s,3H), 3.56 (s,3H), 3.92 (s,3H), 4.45 (d,1H,J = 5.4 Hz), 4.60 (d,1H,J = 5.3 Hz), 7.52 (d,2H,J = 8.3 Hz), 7.98 (d,2H,J = 8.3 Hz). Analysis Calculated for C₁₄H₁₆O₅ : C, 63.62; H, 6.10.
Found: c, 63.14; H, 6.14.

### Step 1c

### (3) (4-(3-Hydroxy-4,4-dimethoxy-butyl)-benzoic acid methyl ester)

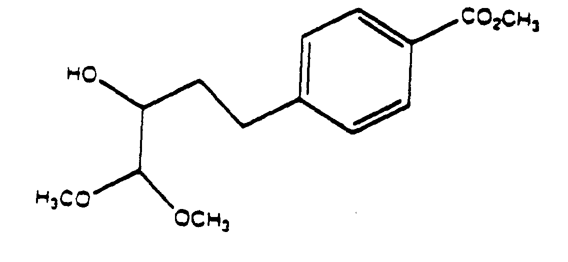

A solution containing 14.37 g (54.38 mmol) of compound (2) and 1.40 g of 5% Pd on carbon in 175 ml of ethanol was hydrogenated under 40 psi Hydrogen on a Parr apparatus. After 2.5 hours, the reaction mixture was filtered and the catalyst washed with ethanol and methanol. After concentrating under reduced pressured, the residue was dissolved in methylene chloride and filtered through a short plug of silica, eluting methylene chloride, then methylene chloride/ethyl acetate (1:1) to remove the residual carbon. In this manner, there was obtained 14.34 g (98%) of the saturated alcohol, compound (3), as a yellow oil.
IR (neat) 3495 (broad), 2953, 1721, 1611, 1437, 1283, 1109, 1080 cm⁻¹.
¹H NMR (CDCl₃) δ 1.72-1.93 (m,2H), 2.75-2.93 (m,2H), 3.39 (s,3H), 3.44 (s,3H), 3.58 (m,1H), 3.90 (s,3H), 4.13 (d,1H,J = 6.1 Hz), 7.29 (d,2H,J = 8.1 Hz), 7.95 (d,2H,J = 8.2 Hz). Analysis calculated for C₁₄H₂₀O₅ · 0.20 H₂O: C, 61.84; H, 7.56.
Found: C, 61.83; H, 757.

### Step 1d

### (4) (4-(3-Methanesulfonyloxy-4,4-dimethoxybutyl)-benzoic acid methyl ester)

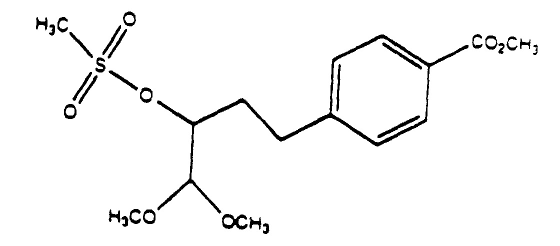

To a stirred solution of 206 mg (.77 mmol) of saturated alcohol, compound (3), and 0.16 ml (1.15 mmol) of triethylamine in 5 ml of methylene chloride at 0°C was added 0.07 ml (.85 mmol) of methanesulfonyl chloride. After 20 minutes at 0°C, another 0.02 ml of methanesulfonyl chloride was added. After 30 minutes more, the reaction mixture was poured into saturated NaHCO₃ solution and extracted twice with methylene chloride. The combined organic layers were dried over MgSO₄ and the solvent was removed under reduced pressure. This material was sufficiently pure for use in the next step. An analytical sample was obtained by flash chromatography on silica eluting methylene chloride/ethyl acetate (20:1). In this manner, there was obtained the mesylate, compound (4), as a colorless oil.
IR (neat), 2949, 2839, 1719, 1611, 1437, 1352, 1283, 1177, 1109, 1078 cm⁻¹.
¹H NMR (CDCl₃) δ 2.04 (m,2H), 2.77-2.91 (m,2H), 3,09 (s,3H), 3.41 (s,3H), 3.45 (s,3H), 3.90 (s,3H), 4.38 (d,1H,J = 5.5 Hz), 4.64 (m,1H), 7.29 (d,2H,J = 8.2 Hz), 7.96 (d,2H,J = 8.2Hz).
Analysis calculated for C₁₅H₂₂O₇S; C, 52.01; H, 6.40; S, 9.26
Found: C, 52.08; H, 6.44; S, 9.25.

### Step 1e

### (5) (4-(3-Methanesulfonyloxy-4-oxobutyl)-benzoic acid methyl ester)

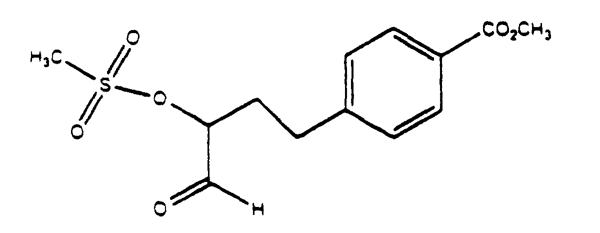

To a stirred solution of 600 mg (1.73 mmol) of dimethyl acetal (meslylate) (compound 4) in 5 ml of methyl chloride at 0°C was added 1 ml of H₂O and 1 ml of Trifluoroacetic acid. The reaction was warmed to room temperature, then refluxed for 24 hours. The cooled reaction mixture was diluted with ethyl acetate and washed twice sequentially with saturated NaCl solution, saturated NaHCO₃ solution, then again with saturated NaCl solution, dried (MgSO₄) and the volatiles were removed under reduced pressure. In this manner, there was obtained the mesylate, compound (5), which was used without purification.
NMR (CDCl₃) δ 2.20 (m,2H), 2.85 (m,2H), 3.17 (s,3H), 3.91 (s,3H), 4.95 (dd,1H,J = 4.2 Hz, 8.4 Hz), 7.29 (d,2H,J = 8.1 Hz), 7.99 (d,2H,J = 8.2 Hz), 9.59 (s,1H).

### Step 1f

### (6) (4-[3-(4-Methoxy-benzylsulfanyl)-4-oxobutyl]-benzoic acid methyl ester

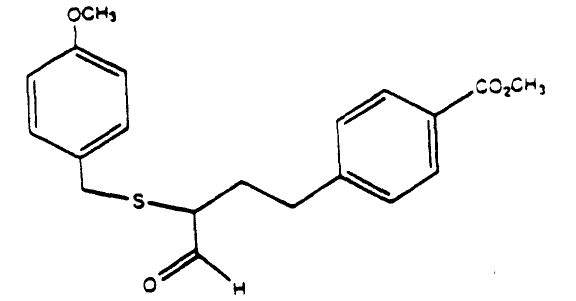

To a stirred solution of 686 mg (2.28 mmol) of the mesylate, compound (5), and 0.40 ml (2.29 mmol) of N,N-Diisopropylethylamine in dimethyl formamide (DMF) was added 0.48 ml (3.44 mmol) of 4-Methoxy-α-toluenethiol. After 3 hours at room temperature, the reaction mixture was poured into 0.5 N HCl and extracted twice with ethyl acetate. The combined organic layers were washed twice with saturated NaCl solution, dried (MgSO₄), and concentrated under reduced pressure. The aldehyde obtained, compound (6), was sufficiently pure to use without further purification in the next step.
IR (KBr) 2930, 1715, 1703, 1611, 1512, 1282, 1244, 1107 cm⁻¹.
¹H NMR (CDCl₃) δ 1.80-2.16 (m,2H), 2.75 (m,2H), 2.99 (m,1H), 3.53 (AB,2H,J = 13.4 Hz), 3.81 (s,3H), 3.90 (s,3H), 6.83 (d,2H,J = 8.5 Hz), 7.12 (d,2H,J = 8.2 Hz), 7.19 (d,2H,J = 8.6Hz), 7.90 (d,2H,J = 8.2 Hz), 9.27 (d,1H,J = 4.2 Hz).

### Step 1g

### (7) (4-[3-[1,3]Dioxolan-2-yl-3-(4-methoxy-benzylsulfanyl)-propyl]-benzoic acid methyl ester

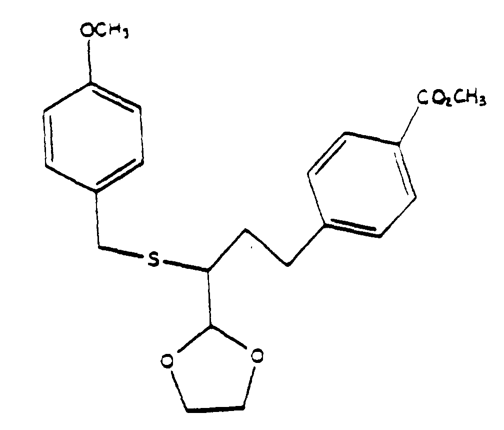

A flask containing 301 mg (.84 mmol) of the aldehyde, compound (6), 94 µl (1.68 mmol) of ethylene glycol and 42 mg (.17 mmol) of Pyridinium p-toluenesulfonate and 30 ml of benzene was heated at reflux removing the generated water with a Dean-Stark trap. After 3 hours, the reaction mixture was poured into saturated NaCl solution and extracted twice with ethyl acetate. The combined organic layers were dried (MgSO₄) and concentrated under reduced pressure. The residue was flash chromatographed on silica, eluting hexanes-ethyl acetate (5:1). In this manner, there was obtained the title compound (7) in an overall 84% yield from the mesylate dimethylacetal, compound (4).
IR (neat), 2949, 2886, 1721, 1611, 1510, 1435, 1279, 1248, 1177, 1111, 1034 cm⁻¹.
¹H NMR (CDCl₃) δ 1.67 (m,1H), 1.99 (m,1H), 2.57 (m,2H), 2.84 (m,1H), 3.72-4.03 (m,6H), 3.81 (s,3H), 3.90 (s,3H), 4.97 (d,1H,J = 4.6 Hz), 6.83 (d,2H,J = 8.6 Hz), 7.09 (d,2H,J = 8.1 Hz), 7.23 (d,2H,J = 8.5 Hz), 7.88 (d,2H,J = 8.1 Hz).
Analysis calculated for C₂₂H₂₆O₅S: C, 65.65; H, 6.51; S, 7.97.
Found: C, 65.72; H, 6.50; S, 8.07.

### Step 1h

### (8) (4-(3-[1,3]Dioxolan-2-yl-3-mercaptopropyl)benzoic acid methyl ester)

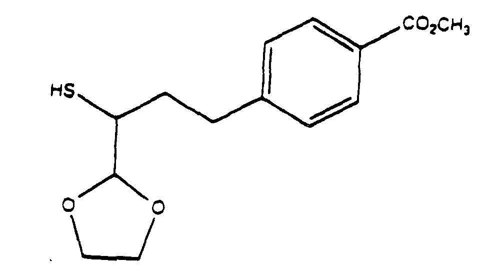

To a stirred solution of 5.70 g (14.16 mmol) of compound (7) and 5.42 g (17.00 mmol) of Mercuric acetate in methylene chloride, cooled to 0°C, was added, dropwise, 5 ml of trifluoroacetic acid. After 3 hours at 0°C, hydrogen sulfide saturated methanol was added and stirring was continued at 0°C for 20 minutes. The reaction mixture was poured into saturated NaCl solution and extracted twice with methylene chloride. The combined organic layers were dried (MgSO₄) and concentrated at reduced pressure. To the crude residue partially suspended in methanol was added 1.07 g (28.28 mmol) of sodium borohydride in portions. After about 30 minutes, the reaction mixture was poured into ethyl acetate and 0.5 N HCl and the layers separated. The metallic mercury that formed in the reaction was washed with ethyl acetate. The combined organic layers were washed with saturated NaCl solution, dried (MgSO4) and concentrated under reduced pressure. The residue was flash chromatographed on silica eluting Hexanes - ethyl acetate (4:1). In this matter, there was obtained 1.99 g (50%) of the thiol, compound (8), as a light yellow oil.
IR (neat), 2951, 2886, 1719, 1611, 1435, 1281, 1179, 1144, 1111 cm⁻¹.
¹H NMR (CDCl₃) δ 1.64 (d,1H,J = 7.8 Hz), 1.74 (m,1H), 2.15 (m,1H), 2.80 (m,2H), 3.00 (m,1H), 3.90 (s,3H), 3.97 (m,4H), 4.91 (d,1H,J = 4.0 Hz), 7.28 (d,2H,J - 8.1 Hz), 7.95 (d,2H,J = 8.1 Hz).
Analysis calculated for C₁₄H₁₈O₄S: C, 59.55; H, 6.14.
Found: C, 59.42; H, 6.41.

### Step 1i

### (9) (4-[3-(2,4_diamino-6-oxo-1,6-dihydro-pyrimidin-5-ylsulfanyl)-3-[1,3]dioxolan-2-yl-propyl]-benzoic acid methyl ester)

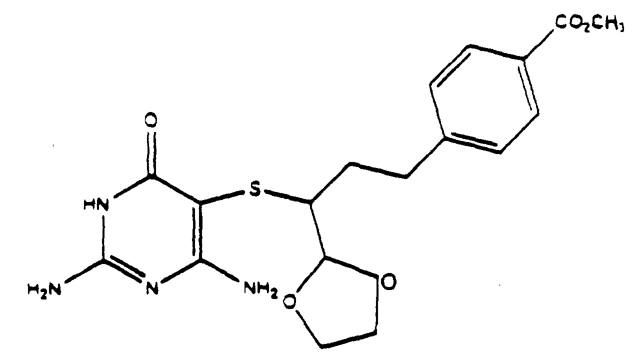

To a stirred solution of 1.91 g (6.76 mmol) of the thiol, compound (8), and 1.39 g (6.78 mmol) of 5-bromo-2,4-diamino-6-oxopyrimidine under argon in degassed N,N-Dimethylformamide was added 1.18 ml (6.77 mmol) of N,N-Diisopropylethylamine. The reaction mixture was heated at 90°C for 3 hours. The cooled reaction mixture was poured into saturated NaCl solution and the precipitate which formed was collected by filtration, washed with water and air dried. The filter cake was slurried in methylene chloride. Hexanes were slowly added and the precipitate was again collected by filtration, washed with hexanes and dried. In this manner, there was obtained 1.91 g (69 %) of the desired dioxolane, compound (9), as an off-white solid: mp 206-208°C with decomposition.
IR (KBr) 3439, 3341, 3154, 1701, 1636, 1591, 1470, 1447, 1287 cm⁻¹.
¹H NMR (DMSO) δ 1.64 (m, 1H), 1.84 (m,1H), 2.58 (m,1H), 2.79 (m,1H), 3.19 (m,1H), 3.82 (s,3H), 3.85 (m,4H), 4.83 (d,1H,J = 4,3 Hz), 6.33 (broad s,4H), 7.33 (d,2H,J = 8.1 Hz), 7.83 (d,2H,J = 8.1 Hz, 10.03 (s,1H).
Analysis calculated for C₁₈H₂₂N₄O₅S: C, 53.19; H, 5.46; N, 13.78; S, 7.89.
Found: C, 52.98; H, 5.53; N, 13.60; S, 7.76.

### Step 1j

### (10) (4-[2-(2-Amino-7-hydroxy-4-oxo-4,6,7,8-tetrahydro-3H-pyrimido[5,4-b][1,4]thiazin-6-yl)-ethyl]-benzoic acid methyl ester)

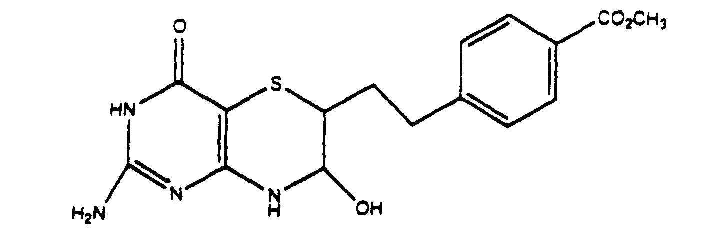

A stirred suspension of 1.20 g (2.85 mmol) of the dioxolane, compound (9), and 4 ml of 2 N HCl in 20 ml of tetrahydrofuran (THF) was heated at reflux for 2.5 hours. The homogeneous solution was poured slowly into saturated NaHCO₃ solution and the precipitate that formed was collected. The filtrate was extracted with ethyl acetate. A precipitate (58 mg) formed between layers and was collected and combined with the first precipitate. The ethyl acetate layer was dried over MgSO₄ and the solvent was removed under reduced pressure. The residue (42 mg) was also combined with the original precipitate. In this manner, there was obtained 984 mg (98%) of the carbinolamine, compound (10), as an orange solid: mp 213-216°C.
IR (KBr) 3351, 3441, 1705, 1638, 1609, 1557, 1470, 1289, 1113, 1020 cm⁻¹.
¹H NMR As a single pair of diastereomers (DMSO) δ 1.39 and 1.96 (m,m,1H), 1.70 (m,1H), 2.56-2.89 (m,3H), 3.82 (s,3H), 4.71 and 4.84 (m,m,1H), 5.37 and 5.40 (d,d,1H,J = 6.6 Hz), 6.06 (s,2H), 7.20 (d,1H,J = 4.5 Hz), 7.31 and 7.36 (d,d,2H,J = 8.1 Hz), 7.86 and 7.88 (d,d,2H,J = 8.0 Hz), 10.16 and 10.19 (s,s,1H).
Analysis calculated for C₁₆H₁₈N₄O₄S · 1.7 H₂O: C, 48.89; H, 5.49; N, 14.26; S, 8.16.
Found: C, 48.78; H, 5.18; N, 14.00; S, 8.03.

### Step 1k

### (11) (4-[2-(Amino-4-oxo-4,6,7,8-tetrahydro-3H-pyrimido[5,4-b][1,4]thiazin-6-yl]-benzoic acid methyl ester

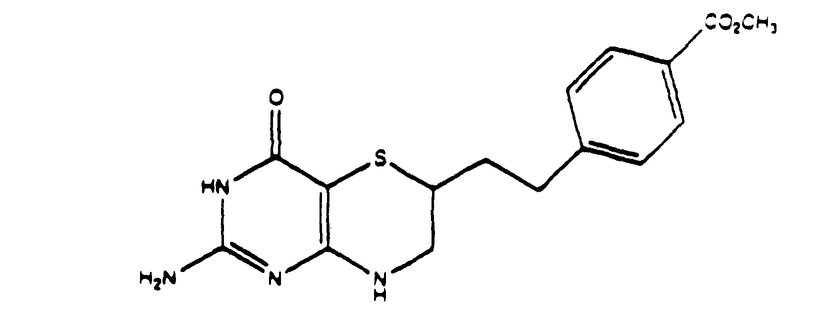

To a O°C suspension of 1.126 g (3.1 mmol) of carbinolamine, compound (10), in tetrahydrofuran (THF) was added 2.3 ml (18.64 mmol) of Boron trifluoride etherate. When the addition was complete, 0.586 g (9.32 mmol) of Sodium cyanoborohydride was added in portions over 5 minutes. After an additional 30 minutes, 5 ml of ammonia saturated methanol was added, the reaction mixture was diluted with ethyl acetate and washed with saturated NaCl solution. The organic layer was dried (MgSO₄) and the solvent removed under reduced pressure. The residue was flash chromatographed on silica eluting methylene chloride/ methanol (9:1). In this manner, there was obtained 542 mg (50%) of the dehydrated ester, compound (11), as an orange solid: mp 245-246°C with decomposition.
IR (KBr) 3358, 2936, 1721, 1644, 1595, 1537, 1447, 1346, 1281 cm⁻¹.
¹H NMR (DMSO) δ 1.72 (m,1H), 1.90 (m,1H), 2.80 (m,3H), 3.22 (m,1H), 3.52 (m,1H), 3.82 (s,3H), 6.00 (s,2H), 6.65 (s,1H), 7.37 (d,2H,J = 8.1 Hz), 7.87 (d,2H,J = 8.1 Hz), 10.05 (s,1H).
Analysis calculated for C₁₆H₁₈N₄O₃S: C, 55.47; H, 5.24; N, 16.17; S, 9.26.
Found: C, 55.31; H, 5.29; N, 16.09; S, 9.17.

### Step 11

### (12) (4-[2-(2-Amino-4-oxo-4,6,7,8-tetrahydro-3H-pyrimido[5,4-b][1,4]thiazin-6-yl)-ethyl]-benzoic acid)

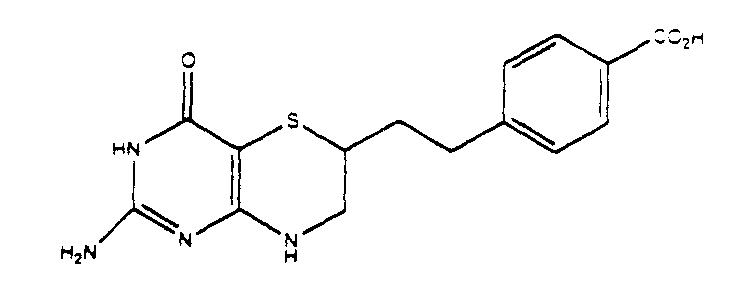

A solution of 530 mg (1.53 mmol) of the ester, compound (11), and 10 ml of IN NaOH was stirred at room temperature for 30 minutes. The homogeneous solution was made slightly acidic (pH 4) with concentration HC1. After cooling in an ice bath, the light orange precipitate was collected by filtration and air dried. It was then suspended in ethanol and the ethanol removed under reduced pressure. In this manner, there was obtained 468 mg (91%) of the acid, compound (12): decomposes > 310°C.
IR (KBr) 3285, 3086, 2928, 1698, 1642, 1611, 1576, 1449, 1348 cm⁻¹.
¹H NMR (DMSO) δ 1.72 (m,1H), 1.89 (m,1H), 2.78 (m,3H), 3.20 (m,1H), 3.48 (m,1H), 6.07 (s,2H), 6.68 (s,1H), 7.33 (d,2H,J = 8.1 Hz), 7.85 (d,2H,J = 8.1 Hz), 10.11 (s,1H), 12.77 (broad s,1H).
Analysis calculated for C₁₅H₁₆N₄O₃S · 1.20 H₂O: c, 50.89; H, 5.24; N, 15.83; S, 9.06.
Found: C, 50.70; H, 4.92; N, 15.58; S, 8.87.

### Step 1m

### (13) (2-[4-[2-(2-Amino-4-oxo-4,6,7,8-tetrahydro-3H-pyrimido [5,4-b][1,4]thiazin-6-yl)-ethyl]-benzoylamino]-pentanedioic acid diethyl ester

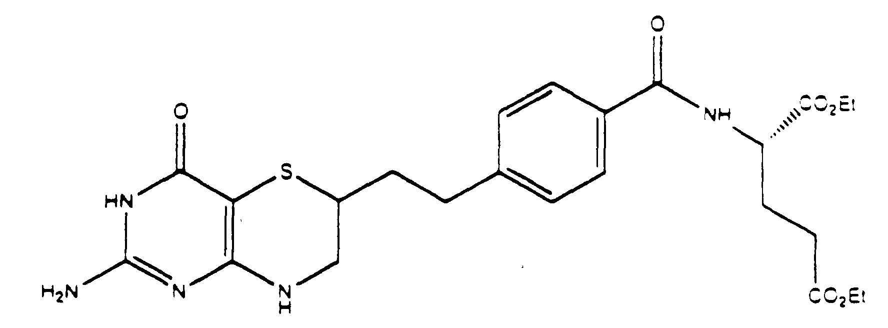

To a stirred solution of 397 mg (1.19 mmol) of the acid, compound (12), 169 mg (1.25 mmol) of 1-Hydroxybenzotriazole hydrate (HOBT), .22 ml (1.25 mmol) of N,N-Diisopropylethylamine and 300 mg (1.25 mmol) of L-Glutamic acid diethyl ester hydrochloride in 15 ml of N,N-dimethylformamide was added 240 mg (1.25 mmol) of 1-(3-Dimethylaminopropyl)-3-ethyl-carbodiimide hydrochloride (EDC). After 18 hours at room temperature, the reaction mixture was poured into ice cold saturated NaCl solution and the precipitate which formed was collected, washed with H₂O and air dried. The precipitate was flash chromatographed on silica eluting methylene chloride/ methanol (9:1). In this manner, there was obtained 357 mg (58%) of the desired product (13) as a light orange solid: mp 132-136°C.
IR (KBr) 3333, 1732, 1645, 1572, 1535, 1449, 1343, 1203, 1020 cm⁻¹.
¹H NMR (DMSO) δ 1.15 (t,3H,J = 7.3 Hz), 1.17 (t,3H,J = 7.3 Hz), 1.72 (m,1H), 1.88-2.10 (m,3H), 2.42 (t,2H,J = 7.4 Hz), 2.79 (m,3H), 3.22 (m,1H), 3.50 (m,1H), 4.02 (q,2H,J = 7.3; 14.5Hz), 4.09 (q,2H,J = 7.2, 14.3 Hz), 4.41 (m,1H), 6.21 (s,2H), 6.74 (s,1H), 7.32 (d,2H,J = 8.0 Hz), 7.80 (d,2H,J = 8.0 Hz),
   8.64 (d,1H,J = 7.41 Hz), 10.24 (s,1H)
Analysis calculated for C₂₄H₃₁N₅O₆S: C, 55.69; H, 6.04; N, 13.53; S, 6.19.
Found: C, 55.41; H, 6.11; N, 13.48; S, 6.12.

### Step 1n

### (14) (2-[4-[2-(2-Amino-4-oxo-4,6,7,8-tetrahydro-3H-pyrimido[5,4-b][1,4]thiazin-6-yl)-ethyl]benzoyl amino)pentanedioic acid)

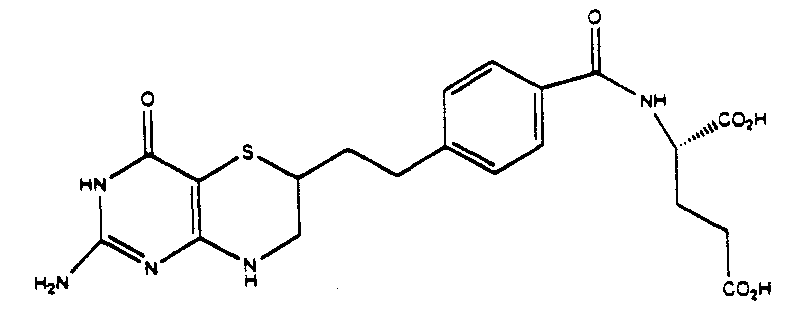

A mixture of 320 mg (.618 mmol) of glutamate, compound (13), and 6 ml of IN NaOH was stirred at room temperature for 3 hours, neutralized with concentrated HCl, then made slightly acidic with 2N HCl. After cooling, the light yellow precipitate was collected and air dried. The filter cake was taken up in ethanol/acetonitrile and any residual H₂O was azeotrophed off. In this manner, there was obtained 220 mg (77%) of the diacid, compound (14): mp: 188-190°C.
IR (KBr) 3348 (broad), 2930, 1717, 1642, 1539, 1505, 1348 cm⁻¹.
¹H NMR (DMSO) δ 1.71 (m,1H), 1.92 (m,2H), 2.08 (m,1H), 2.34 (t,2H,J = 7.4 Hz), 2.79 (m,3H), 3.20 (m,1H), 3.55 (m,1H), 4.38 (m,1H), 6.07 (s,2H), 6.68 (s,1H), 7.31 (d,2H,J = 8.1 Hz), 7.80 (d,2H,J = 8.2 Hz), 8.53 (d,1H,J = 7.7 Hz), 10.11 (s,1H), 12.40 (broad s,2H).
Analysis calculated for C₂₀H₂₃N₅O₆S · 1.5 H₂O: C, 49.17; H, 5.36; N, 14.34; S, 6.56.
Found: C, 48.77; H, 4.97, N, 14.07; S, 6.54.

### EXAMPLE 2

### Biological and Biochemical Evaluation

### In Vitro Testing

Cellular growth in the presence of the compounds according to the present invention was assessed using the L1210 murine leukemia (ATCC CCL 219) cell line. The cell line was maintained in RPMI 1640 medium containing 5% heat-inactivated fetal bovine serum without antibiotics.

IC₅₀ values were determined in 160 microliter microcultures containing 1500 (L1210) cells established in 96 well plates in growth medium supplemented with 50 IU/mL penicillin and 50 mcg/mL streptomycin. Growth was measured over 3 days of continuous exposure to varying concentrations of the test compound (14) added 4 hours after initial cell plating by the MITT-tetrazolium reduction assay of Mosmann (Immunol. Meth. 65, 55-63 (1983)), modified according to Alley et al. (Cancer Res. 48, 589-601 (1988)). Water insoluble derivatives were dissolved in DMSO and diluted to a final concentration of 0.5% solvent in cell cultures.

### Determination of Inhibition Constants for Gar Transformylase

GAR transformylase inhibition constant was measured by the method of Cleland (Biochem. Biophys. Acta 67, 173-187 (1963)). Assays were done at 22°C and initiated by addition of enzyme using the spectrophotometric assay of Young et al. (Biochemistry 23, 3979-3986 (1984)) and monitoring the reaction at 295 nm. The GAR transformylase domain of the human enzyme was used. The variable substrate was 10-formyl-5-8-dideazafolate at concentrations of 0.83 µM, 1.25 µM, 2.5 µM and 5 µM while the other substrate, GAR (glycinamide ribonucleotide), was held constant at 20 µM. The assay mix contained 20 mM Hepes pH 7.5, 20 *µ*M GAR, and variable amounts of 10-formyl-5, 8-dideazafolate and inhibitor. For the inhibitor (14), five concentrations were used ranging from 0 to approximately 3Ki. The data were plotted as the velocity of the reaction versus the reciprocal of the 10-formyl-5, 8-dideaza-folate concentration. The inhibition constant was measured from a replot of the slopes of these lines obtained for each concentration of inhibitor versus the inhibitor concentration.

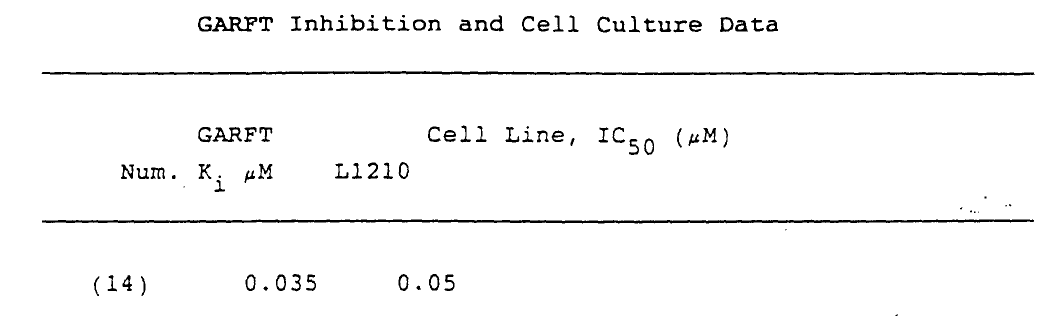

The compounds and intermediates of the invention contain one or more chiral centers. The invention encompasses racemic mixtures, mixtures of diastereomers, and optically active compounds, such as compounds essentially free of other optical isomers, which optically active compounds can be obtained by means well-known to those skilled in the art.

## Claims

1. A compound of the formula (I): 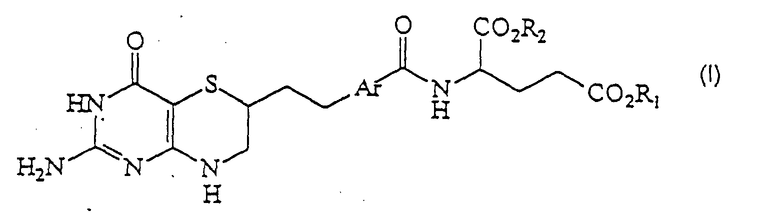 wherein
Ar is a monocyclic carbocyclic or heterocyclic ring;
and
R₁ and R₂ are independently hydrogen or a C₁₋₆ alkyl group; or a pharmaceutically acceptable salt thereof.

2. The compound or salt according to claim 1, wherein Ar is phenylene.

3. The compound or salt according to claim 1 or 2, wherein R₁ and R₂ are each hydrogen.

4. d-(2-[4-[2-(2-Amino-4-oxo-4,6,7,8-tetrahydro-3H-pyrimido[5,4-b][1,4]thiazin-6-yl)-ethyl]-benzoylamino]-petanedioic acid diethyl ester).

5. d-(2-[4-[2-(2-Amino-4-oxo-4,6,7,8-tetrahydro-3H-pyrimido[54-b][1,4]thiazin-6-yl)-ethyl]-benzoylamino]-petanedioic acid).

6. The compound or salt according to any of claims 1 to 5, wherein the compound is optically active.

7. An antiproliferative composition comprising the compound according to any of claims 1 to 6 in combination with a pharmaceutically acceptable carrier.

8. Use of the compound according to any of claims 1 to 6 for the preparation of a medicament for inhibiting the growth and proliferation of the cells of microorganisms or of higher organisms in a host.

9. Use of the compound according to any of claims 1 to 6 for the preparation of a medicament for inhibiting GARFT.

## Patentansprüche

1. Verbindung der Formel (I): 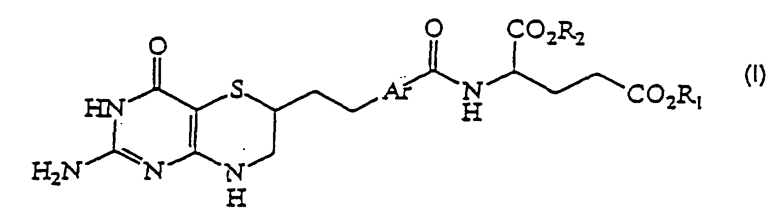 worin
Ar ein monocyclischer, carbocyclischer oder heterocyclischer Ring ist; und
R₁ und R₂ unabhängig voneinander Wasserstoff oder eine C₁₋₆-Alkylgruppe bedeuten;
oder ein pharmazeutisch annehmbares Salz davon.

2. Verbindung oder Salz nach Anspruch 1, worin Ar Phenylen ist.

3. Verbindung oder Salz nach Anspruch 1 oder 2, worin R₁ und R₂ jeweils Wasserstoff sind.

4. d-(2-[4-[2-(2-Amino-4-oxo-4,6,7,8-tetrahydro-3H-pyrimido[5,4-b][1,4]thiazin-6-yl)- )-ethyl]-benzoylamino]-petandisäure-diethylester).

5. d-(2-[4-[2-(2-Amino-4-oxo-4,6,7,8-tetrahydro-3H-pyrimido[5,4-b][1,4]thiazin-6-yl)-ethyl]-benzoylamino]-petandisäure).

6. Verbindung oder Salz nach einem der Ansprüche 1 bis 5, worin die Verbindung optisch aktiv ist.

7. Proliferationshemmende Zusammensetzung, umfassend die Verbindung nach einem der Ansprüche 1 bis 6 in Kombination mit einem pharmazeutisch annehmbaren Träger.

8. Verwendung der Verbindung gemäß einem der Ansprüche 1 bis 6 zur Herstellung eines Medikaments zur Hemmung des Wachstums und der Proliferation von Zellen von Mikroorganismen oder höheren Organismen in einem Wirt.

9. Verwendung der Verbindung nach einem der Ansprüche 1 bis 6 zur Herstellung eines Medikaments zur Inhibierung von GARFT.

## Revendications

1. Composé de formule (I): 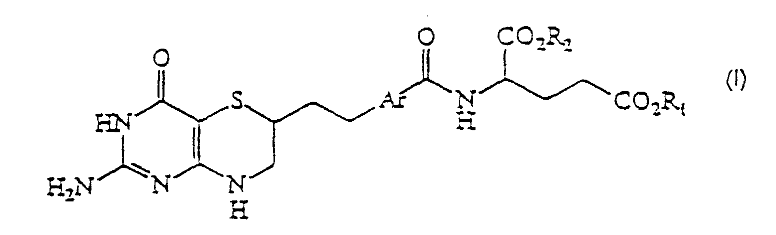 dans laquelle:
Ar est un noyau monocyclique carbocyclique ou hétérocyclique;
et
R₁ et R₂ sont, indépendamment l'un de l'autre, un atome d'hydrogène ou un groupe alkyle en C₁ à C₆; ou un de ses sels acceptables sur le plan pharmaceutique.

2. Composé ou sel selon la revendication 1, dans lequel Ar est le phénylène.

3. Composé ou sel selon la revendication 1 ou 2, dans lequel R₁ et R₂ sont chacun un atome d'hydrogène.

4. L'ester diéthylique de l'acide d-(2-[4-[2-(2-Amino-4-oxo-4,6,7,8-tétrahydro-3H-pyrimido [5,4-b] [1, 4]thiazine-6-yl)-éthyl] -benzoylamino]-pentanedioïque.

5. L'acide d-(2-[4-[2-(2-Amino-4-oxo-4,6,7,8-tétrahydro-3H-pyrimido[5,4-b][1,4]thiazine-6-yl)-éthyl]-benzoylamino]-pentanedioïque.

6. Composé ou sel selon l'une quelconque des revendications 1 à 5, dans lequel le composé est optiquement actif.

7. Composition antiproliférative comprenant le composé selon l'une quelconque des revendications 1 à 6 en combinaison avec un véhicule acceptable sur le plan pharmaceutique.

8. Utilisation du composé selon l'une quelconque des revendications 1 to 6 pour la préparation d'un médicament pour inhiber la croissance et la prolifération cellulaire de micro-organismes ou d'organismes supérieurs chez un hôte.

9. Utilisation du composé selon l'une quelconque des revendications 1 to 6 pour la préparation d'un médicament destiné à inhiber GARFT.
